Europäisches Patentamt

European Patent Office

Office européen des brevets

(19)

(11) Numéro de publication : **0 371 844 B1**

(12) **FASCICULE DE BREVET EUROPEEN**

(45) Date de publication du fascicule du brevet :
29.07.92 Bulletin 92/31

(51) Int. Cl.⁵ : **A61K 7/48,** A61K 31/455,
A61K 31/355

(21) Numéro de dépôt : **89403150.9**

(22) Date de dépôt : **16.11.89**

(54) **Utilisation du nicotinate d'alpha-tocophérol, de benzyle, de xanthinol ou d'hexyle ou de l'acétate d'alpha-tocophérol dans une composition cosmétique ou pharmaceutique à action amaigrissante.**

(30) Priorité : **17.11.88 LU 87390**

(43) Date de publication de la demande :
**06.06.90 Bulletin 90/23**

(45) Mention de la délivrance du brevet :
**29.07.92 Bulletin 92/31**

(84) Etats contractants désignés :
**AT BE CH DE FR GB IT LI NL SE**

(56) Documents cités :
**EP-A- 0 220 825**
**FR-A- 2 105 254**
**GB-A- 2 002 232**
**GB-A- 2 002 233**
**GB-A- 2 166 954**

(56) Documents cités :
**PATENT ABSTRACTS OF JAPAN, vol. 12, no. 47 (C-475)[2894], 12 février 1988; & JP-A-62 195 316 (KAO CORP.)**
**PATENT ABSTRACTS OF JAPAN, vol. 10, no. 339 (C-385)[2395], 15 novembre 1986; & JP-A-61 143 311 (POLA CHEM. IND.)**

(73) Titulaire : **L'OREAL**
**14, Rue Royale**
**F-75008 Paris (FR)**

(72) Inventeur : **Soudant, Etienne**
**14, rue Léon Bernard**
**F-94260 Fresnes (FR)**

(74) Mandataire : **Stalla-Bourdillon, Bernard et al**
**CABINET NONY & CIE 29, rue Cambacérès**
**F-75008 Paris (FR)**

EP 0 371 844 B1

## Description

La présente invention a pour objet l'utilisation de nicotinate d' $\alpha$-tocophérol, de benzyle, de xanthinol ou d'hexyle ou de l'acétate d'$\alpha$-tocophérol en tant que seule (s) substance (s) à activité caféine-like dans une composition à action amaigrissante.

Plus particulièrement, la présente invention est destinée à améliorer l'esthétique et notamment l'affinement de la silhouette par amaigrissement.

Comme ceci est maintenant bien connu, la cellulite est constituée par l'accumulation locale de graisse et d'eau emprisonnées dans une gangue à compartiments plus ou moins étanches. Cette gangue est constituée d'éléments de la substance fondamentale et plus spécialement de protéoglycanes qui sont des substances polymériques.

De façon à libérer les graisses emprisonnées et l'eau liée aux substances polymériques, il a été proposé d'utiliser, par application locale, des compositions à base d'enzymes capables de dépolymériser les protéoglycanes. Ces enzymes sont des mucopolysaccharidases et plus particulièrement l'hyaluronidase, la thiomucase et l' $\alpha$-mucase.

Parmi les méthodes de stimulation de la lipolyse, la plus connue et la plus utilisée est celle qui consiste à inhiber la phosphodiesterase afin d'empêcher ou du moins limiter la vitesse de dégradation de l'AMP cyclique. En effet, la phosphodiestérase détruit l'AMP cyclique en le transformant en 5' AMP de telle sorte qu'il ne peut jouer le rôle d'activateur de la lipolyse.

Il importe donc d'inhiber l'action de la phosphodiestérase de façon à avoir un taux élevé d'AMP cyclique au niveau des adipocytes dans le but de stimuler l'activité lipolytique.

Parmi les différents inhibiteurs de phosphodiestérase qui ont été préconisés comme amaigrissant, on peut en particulier mentionner les bases xanthiques et plus particulièrement la théophylline, la caféine et la théobromine.

Dans la même optique, il a également été préconisé l'utilisation de certains extraits végétaux oléosolubles qui selon un mécanisme différent, peuvent également agir comme amaigrissant. Parmi ces extraits végétaux on peut notamment mentionner ceux de lierre grimpant, d'arnica, de romarin, de souci, de sauge, de ginseng, de millepertuis, de fragon, d'ulmaire et d'orthosiphon ainsi que les mélanges de tels extraits végétaux.

Dans la pratique, ces substances à action amaigrissante ou lipolytique en particulier les inhibiteurs de phosphodiestérase et les extraits végétaux oléosolubles, sont le plus souvent associés à au moins une substance à action hyperémiante ou vasodilatatrice permettant de favoriser la microcirculation.

Parmi les substances présentant une telle action, parfois désignées sous la dénomination d'agents rubéfiants, on peut notamment mentionner les extraits de capsicum, les sels de l'acide nicotinique tels que le nicotinate de triéthanolamine, les esters de l'acide nicotinique tels que par exemple le nicotinate de méthyle, d'éthyle, d'hexyle, de phényle et de benzyle ainsi que le nicotinate d' $\alpha$-tocophérol.

Ces substances à action rubéfiante dont certaines provoquent un léger échauffement de la peau, permettent le drainage de l'eau emprisonnée dans les tissus cellulitiques et favorisent l'évacuation hors de ces tissus des acides gras lorsqu'il y a eu lipolyse et seulement après. De plus l'échauffement éventuellement provoqué par ces substances favorise la diffusion des principes actifs.

Après d'importantes études, on vient maintenant de constater de façon tout à fait surprenante que certaines de ces substances, en plus de leurs activités connues sur la microcirculation présentaient également une excellente action lipolytique.

Cette nouvelle propriété qui n'avait jamais été mise en évidence jusqu'à présent permet donc de traiter les effets disgracieux de la cellulite et des surcharges graisseuses localisées, sans avoir recours à la présence conjointe d'une substance à action lipolytique du type caféine.

La présente invention a donc pour objet une nouvelle utilisation de nicotinate d' $\alpha$-tocophérol, de benzyle, de xanthinol ou d'hexyle, ou de l'acétate d'$\alpha$-tocophérol en tant que substance(s) lipolytique (s) à activité caféine-like pour la préparation d'une composition cosmétique ou pharmaceutique à action amaigrissante, ladite composition étant destinée à combattre la cellulite et les surcharges graisseuses localisées, celle-ci étant exempte de toute autre substance à activité caféine-like connue.

Selon l'invention la substance lipolytique est généralement présente à une concentration comprise entre 0,1 et 10% en poids et de préférence entre 0,5 et 5% en poids par rapport au poids total de la composition.

L'administration peut être effectuée par voie entérale, parentérale, rectale ou topique.

Par voie entérale, les médicaments peuvent se présenter sous forme de comprimés, de gélules, de dragées, de sirops, de suspensions, de solutions , de poudres, de granulés ou d'émulsions.

Par voie parentérale, les compositions peuvent se présenter sous forme de solutions ou suspensions pour perfusion ou pour injection. Par voie rectale, les compositions se présentent sous forme de suppositoires.

Administrées par voie topique, ces compositions peuvent se présenter sous différentes formes en particulier sous forme anhydre telle que par exemple une huile ou un baume ou encore sous forme d'une émulsion huile-dans-l'eau ou eau-dans-l'huile ayant l'aspect d'une crème ou d'un lait.

Lorsque la composition est sous forme anhydre, l'excipient peut être une huile végétale ou animale, une huile minérale ou encore une huile synthétique ou des mélanges de ces huiles.

Parmi les huiles végétales ou animales, modifiées ou non, on peut citer par exemple l'huile d'amande douce, l'huile d'avocat, l'huile de ricin, l'huile d'olive, l'huile de jojoba, le perhydrosqualène, l'huile de calophyllum, la lanoline et ses dérivés, l'huile de tournesol, l'huile de germes de blé, l'huile de sésame, l'huile d'arachide, l'huile de pépins de raisin, l'huile de soja, l'huile de colza, l'huile de carthame, l'huile de coco, l'huile de maïs, l'huile de noisette, le beurre de karité, la graisse de shorea robusta, l'huile de palme et l'huile de noyau d'abricot.

Parmi les huiles minérales on peut citer par exemple l'huile de vaseline et parmi les huiles synthétiques, les palmitates d'éthyle et d'isopropyle, les myristates d'alkyle tel que le myristate d'isopropyle, de butyle, de cétyle, le stéarate d'hexyle, les triglycérides des acides octanoïque et décanoïque (par exemple le produit vendu sous la dénomination de "MIGLYOL" par la Société DYNAMIT NOBEL), le ricinoléate de cétyle, l'octanoate de stéaryle (huile de purcellin) et le polyisobutène hydrogéné ainsi que des cires telles que l'ozokérite.

L'excipient gras peut également contenir certains composés considérés comme des produits gras à savoir des alcools à longue chaîne tels que l'alcool cétylique, l'alcool stéarylique, l'alcool myristique, l'alcool hydroxystéarylique l'alcool oléique ou l'alcool isostéarylique.

Lorsque les compositions se présentent sous forme d'une émulsion, la phase grasse des émulsions représente de 10 à 80% en poids, la phase eau de 15 à 80% et l'agent émulsionnant de 5 à 30% en poids par rapport au poids total de l'émulsion.

Les compositions selon l'invention peuvent également contenir divers ingrédients conventionnels tels que par exemple des tensio-actifs, des polymères, des huiles de silicone, des parfums, des colorants, des édulcorants, des anti-oxydants ou des agents conservateurs.

Il convient de remarquer que le nicotinate d'hexyle du fait de ses propriétés rubéfiantes prononcées permet de réaliser des compositions thermo-amaigrissantes particulièrement agréables à l'application.


<u>Etude de l'effet lipolytique des substances actives sur des cellules adipeuses en culture.</u>


Les cellules adipeuses peuvent, contrairement aux adipocytes isolés du tissu adipeux qui cessent d'être viables en quelques heures, être maintenues jusqu'à trois semaines sans perte de viabilité (Ailhaud G., Mol. Cell Biochem. 49.17-31, 1982).

L'étude a été réalisée sur des cellules de Rat 0b 17 différenciées cultivées dans des conditions parfaitement définies (Gaillard D. et al, Biochim. Biophys. Acta, <u>846</u>, 185-91, 1985 et Doglio A. et al, Biochem. J. <u>238</u>, 123-129, 1986).

Les expériences de lipolyse ont été conduites après exposition des cellules à l'acétate marqué ou $^{14}C$ (2 périodes de 48 h en présence de 0,5 μ Ci/boîte). Dans des conditions d'état stationnaire, la radioactivité est retrouvée principalement dans les acides gras des triglycérides (Negrel R et al, Proc. Natl-Acad. Sci. USA, <u>75</u>, 6054-6058, 1978 et Forest C. et al, Exp. Cell Res, <u>168</u>, 218-232, 1987).

Les essais de lipolyse ont été réalisés à 37°C en fonction du temps. Il est prévu au minimum 2 boîtes par condition et 4 points de cinétique au minimum par boîte.

Les substances actives ont été testées seules et associées avec de la caféine. Les concentrations sont sélectionnées par des tests de viabilité (exclusion du Bleu TRYPAN et relarguage spontané de la lactate deshydrogénase cytoplasmique)

Dans un premier temps la concentration la plus élevée de chaque molécule compatible avec le maintien de la viabilité est utilisée pour des essais de lipolyse.

Dans un second temps les molécules sont étudiées en dose-réponse (7 concentrations par substance active, seule ou associée, aux différentes puissances de 10 entre $10^{-9}$ et $10^{-3}$M).

Dans tous les cas les activités de lipolyse des substances actives sont comparées à celles obtenues en présence d'une concentration maximalement stimulante de deux molécules de référence : un β-adrénergique (isoprénaline) et caféine à une concentration de $10^{-1}$M (dose maximale non cytotoxique).

Les résultats sont reportés dans le tableau suivant :

| X | Pourcentage de stimulation de la lipolyse de X seul | Pourcentage de stimulation de la lipolyse de X + caféine |
|---|---|---|
| Nicotinate d' α-tocophérol | 100 % | 46 % |
| Nicotinate de benzyle | 100 % | 45 % |
| Nicotinate d'hexyle | 35 % | 35 % |
| Nicotinate de xanthinol | 100 % | 51 % |
| Acétate d'α-tocophérol | 50 % | 35 % |
| Nicotinate de méthyle | ∿ 0 % | 35 % |
| Caféine | 35 % | – |

Remarques : 100 % correspond au maximum d'effet de l'isoprénaline (β-agoniste) et au maximum d'effet non cytotoxique de la caféine.

Les valeurs données dans le tableau correspondent à l'effet maximum.

Comme on peut le constater et de façon tout à fait surprenante, le nicotinate d'α-tocophérol, le nicotinate de benzyle, le nicotinate d'hexyle, le nicotinate de xanthinol et l'acétate d'α-tocophérol se sont avérés présenter une excellente action lipolytique supérieure à celle de la caféine pour la plupart de ces substances.

Par contre, aucune action lipolytique n'a pu être observée à l'égard du nicotinate de méthyle. Ces études montrent par ailleurs qu'aucune synergie notable n'est observée et qu'en fait la caféine lorsqu'elle est associée à ces substances inhibe l'action lipolytique.

Si le nicotinate d'hexyle présente une action lipolytique identique à celle de la caféine, il possède néanmoins l'avantage d'associer des propriétés rubéfiantes permettant la réalisation de compositions thermo-amaigrissantes.

On va maintenant donner à titre d'illustration plusieurs exemples de compositions à action amaigrissante contenant comme seule (s) substance (s) active (s) amaigrissante (s) à activité caféine-like du nicotinate d'α-tocophérol, de benzyle, de xanthinol ou d'hexyle ou de l'acétate d'α-tocophérol.

EXEMPLES DE COMPOSITIONS

EXEMPLE 1

Baume amincissant

- Nicotinate d' α -tocophérol........... 5%
- Ozokérite naturel.................... 20%
- Huile de purcellin liquide........... 10%
- Vaseline blanche..................... 15%
- Conservateur......................... 0,2%
- Antioxydant.......................... 0,3%
- Huile de vaseline qsp................ 100%

EXEMPLE 2

Huile corporelle thermo-amincissante

- Nicotinate d'hexyle.................. 2%
- Nicotinage de xanthinol ............. 2%
- "Miglyol 812" (Triglycérides d'acides
  gras) de la Société DYNAMIT NOBEL..... 15%
- Palmitate d'isopropyle............... 10%
- Huile d'amande douce................. 5%

- Conservateur......................... 0,2%
- Parfum............................... 1%
- Antioxydant.......................... 0,1%
- Huile de vaseline qsp................ 100%

EXEMPLE 3

Mousse thermo-amincissante

```
- Nicotinate d'hexyle.................    1,5%
- Polymère cationique "CELQUAT L-200" de
  la Société NATIONAL STARCH...........    0,3%
- Tensio-actif (TWEEN 20)..............     2%
- Nonylphénol oxyéthyléné (12 moles
  d'oxyde d'éthylène)..................    10%
- Alcool éthylique.....................    25%
- Glycérine............................     5%
- Conservateur.........................    0,3%
- Eau qsp..............................   100%
```

Cette composition (95g) est conditionnée dans un récipient aérosol en présence de 5g de butane en tant qu'agent propulseur.

EXEMPLE 4

Emulsion huile-dans-l'eau amincissante

```
- Nicotinate d' α -tocophérol..........     2%
- Huile de silicone....................    10%
- Perhydrosqualène.....................    25%
- Monostéarate de glycérol-Stéarate de
  Polyéthylèneglycol 100 (50-50) commer-
  cialisé sous la dénomination "Arlacel 165"
  par la Société ATLAS.................     6%
- Polysorbate 60.......................     2%
- Alcool cétylique.....................    1,2%
- Acide stéarique......................    2,5%
- Triéthanolamine......................    0,1%
- Conservateur.........................    0,3%
- Antioxydants ........................    0,3%
- Eau qsp..............................   100%
```

EXEMPLE 5

Gel limpide amincissant

| | |
|---|---|
| – Nicotinate de benzyle ............... | 0,5% |
| – Nonylphénol oxyéthyléné (à 12 moles d'oxyde d'éthylène).................... | 5% |
| – Acide polyacrylique réticulé vendu sous la dénomination de "Carbopol 940" par la Société GOODRICH.................... | 1% |
| – Alcool éthylique...................... | 30% |
| – Triéthanolamine....................... | 0,3% |
| – Glycérine............................. | 3% |
| – Parfum................................ | 0,3% |
| – Conservateur.......................... | 0,3% |
| – Eau qsp............................... | 100% |

Les compositions des exemples 2 et 3 par application sur la peau provoquent un léger échauffement dû à l'action rubéfiante du nicotinate d'hexyle.

**Revendications**

1. Utilisation du nicotinate d'$\alpha$-tocophérol,de benzyle, de xanthinol ou d'hexyle ou de l'acétate d'$\alpha$-tocophérol ou leur mélange, en tant que substance (s) lipolytique (s) pour la préparation d'une composition pharmaceutique ou cosmétique à action amaigrissante, ladite composition étant destinée à combattre la cellulite et les surcharges graisseuses localisées, ladite composition étant exempte de toute autre substance lipolytique à activité caféine-like.

2. Utilisation selon la revendication 1, caractérisée par le fait que la composition contient la substance lipolytique à une concentration comprise entre 0,1 et 10% en poids et de préférence entre 0,5 et 5% en poids par rapport au poids total de la composition.

3. Utilisation selon l'une des revendications 1 ou 2, caractérisée par le fait que la composition est une composition pharmaceutique se présentant sous forme de comprimés, de granulés, de gélules, de dragées, d'un sirop, d'une suspension, d'une solution, d'une poudre, d'une émulsion ou d'un suppositoire.

4. Utilisation selon l'une des revendications 1 ou 2, caractérisée par le fait que la composition est une composition cosmétique se présentant sous forme d'une huile ou d'un baume ou sous forme d'une émulsion huile-dans-l'eau ou eau-dans-l'huile ayant l'aspect d'une crème ou d'un lait.

5. Utilisation selon l'une quelconque des revendications précédentes, caractérisée par le fait que la composition contient en outre au moins un ingrédient pharmaceutique ou cosmétique conventionnel tel que par exemple un tensio-actif, un polymère, une huile de silicone, un parfum, un colorant, un édulcorant un antioxydant ou un agent conservateur.

**Claims**

1. Use of $\alpha$-tocopherol nicotinate, benzyl nicotinate, xanthinol nicotinate, hexyl nicotinate or $\alpha$-tocopherol acetate, or a mixture thereof, as lipolytic substance(s) for the preparation of a pharmaceutical or cosmetic composition having a slimming action, the said composition being intended for combating cellulite and localised accumulations of excess fat, the said composition being free from all other lipolytic substances having caffeine-like activity.

2. Use according to Claim 1, characterised in that the composition contains the lipolytic substance at a concentration of between 0.1 and 10 % by weight, and preferably between 0.5 and 5 % by weight, relative to the total weight of the composition.

3. Use according to one of Claims 1 and 2, characterised in that the composition is a pharmaceutical composition which takes the form of tablets, granules, hard gelatin capsules, dragees, a syrup, a suspension, a solution, a powder, an emulsion or a suppository.

4. Use according to one of Claims 1 and 2, characterised in that the composition is a cosmetic composition which takes the form of an oil or a balm or the form of an oil-in-water or water-in-oil emulsion having the appearance of a cream or a milk.

5. Use according to any one of the preceding claims, characterised in that the composition contains, in addition, at least one conventional pharmaceutical or cosmetic ingredient, such as, for example, a surfactant, a polymer, a silicone oil, a perfume, a colouring, a sweetener, an antioxidant or a preservative.

**Patentansprüche**

1. Verwendung von $\alpha$-Tocopherol-, Benzyl-, Xanthinol- oder Hexyl-Nicotinat oder $\alpha$-Tocopherol-Acetat oder deren Mischung als lipolytische Substanz(en) zur Herstellung eines kosmetischen oder pharmazeutischen Abmagerungsmittels zur Bekämpfung der Cellulitis und von lokalen Fettübergewichten, wobei die Zusammensetzung vollständig frei von anderen lipolytischen Substanzen mit einer koffeinähnlichen Wirkung ist.

2. Verwendung nach Anspruch 1, dadurch gekennzeichnet, daß die Zusammensetzung die lipolytische Substanz in einer Konzentration zwischen 0,1 und 10 Gew.-% und vorzugsweise zwischen 0,5 und 5 Gew.-%, bezogen auf das Gesamtgewicht der Zusammensetzung, enthält.

3. Verwendung nach einem der Ansprüche 1 oder 2, dadurch gekennzeichnet, daß die Zusammensetzung eine pharmazeutische Zusammensetzung in Form von Tabletten, eines Granulates, Kapseln, Dragees, eines Sirups, einer Suspension, einer Lösung, eines Puders, einer Emulsion oder in Form von Suppositorien vorliegt.

4. Verwendung nach einem der Ansprüche 1 oder 2, dadurch gekennzeichnet, daß die Zusammensetzung eine kosmetische Zusammensetzung in Form eines Öls oder einer Salbe oder in Form einer Öl-in-Wasser- oder Wasser-in-Öl-Emulsion mit dem Aussehen einer Creme oder einer Milch vorliegt.

5. Verwendung nach einem der vorhergehenden Ansprüche, dadurch gekennzeichnet, daß die Zusammensetzung mindestens einen andern üblichen pharmazeutischen oder kosmetischen Bestandteil wie z. B. ein Detergens, ein Polymer, ein Silikonöl, ein Parfum, einen Farbstoff, einen Süßstoff, ein Antioxidans oder ein Konservierungsmittel enthält.